# EUROPEAN PATENT SPECIFICATION

(11) **EP 2 257 512 B1**
(45) Date of publication and mention of the grant of the patent: **27.06.2012**
(21) Application number: 08868518.5
(22) Date of filing: 22.12.2008
(51) Int. Cl.: C07C 17/25, C07C 17/354, C07C 17/383, C07C 19/08, C07C 21/18

(54) **PROCESS FOR PRODUCING 1,1,1,2-TETRAFLUOROPROPENE**
VERFAHREN ZUR HERSTELLUNG VON 1,1,1,2-TETRAFLUORPROPEN
PROCÉDÉ DE FABRICATION DE 1,1,1,2-TÉTRAFLUOROPROPÈNE

(30) Priority: 27.12.2007 US 17052 P
(43) Date of publication of application: 08.12.2010
(60) Divisional application: 10194845.3
(73) Proprietor: Arkema France, 92700 Colombes (FR)
(72) Inventor: TAKAHASHI, Kazuhiro, Settsu-shi Osaka 566-8585 (JP); SHIBANUMA, Takashi, Settsu-shi Osaka 566-8585 (JP)
(74) Representative: Dang, Doris
(86) International application number: PCT/JP2008/073902
(87) International publication number: WO 2009/084703

(56) References cited:
- US-A- 4 086 407
- US-A1- 2007 179 324

## Description

### Technical Field

The present invention relates to a process for producing 1,1,1,2-tetrafluoropropene.

### Background Art

CFCs (chlorofluorocarbons) and HCFCs (hydrochlorofluorocarbons) were conventionally used as refrigerants. Since these substances can cause depletion of the ozone layer, HFCs (hydrofluorocarbons), particularly HFC-125 (pentafluoroethane) and HFC-32 (difluoromethane) have widely been used as substitute refrigerants. However, HFC-125 and HFC-32 are potent global warming substances and cause concern of diffusion of these substances to adversely affect the global warming. Although these substances are recovered from disused apparatuses for the purpose of preventing their diffusion and consequently the global warming, all of these substances cannot be recovered. Also, diffusion caused by leaking or the like cannot be neglected. Then, CO₂ and hydrocarbon compounds are studied to be used as other substitute refrigerants. However, the CO₂ refrigerant has poor efficiency and requires a larger device, so that there are a lot of problems in reducing overall greenhouse gas emission including consumption energy of the device. Also, the hydrocarbon compounds have problems about safety due to their high combustibility.

Recently, intense interest has been shown towards, as a substitute refrigerant being able to solve these problems, 1,1,1,2-tetrafluoropropene (or 2,3,3,3-tetrafluoropropene, CF₃CF=CH₂, hereinafter also referred to as "HFC-1234yf"), which is a HFC of an olefin having a low warming coefficient.

Some processes for producing HFC-1234yf have hitherto been disclosed. Patent Document 1 listed below describes a process of using CF₃CH=CH₂ as a raw material, halogenating it and then subjecting to a dehydrofluorination reaction to obtain HFC-1234yf. Patent Document 2 listed below describes a process of hydrogenating a fluorinated olefin having 3 to 6 carbon atoms through at least two reaction stages to give an alkane, and dehydrofluorinating the alkane to produce a fluorinated olefin such as HFC-1234yf. Patent Document 3 listed below describes a process of directly obtaining HFC-1234yf from a mixture of CF₃CF₂CHCl₂ (HCFC-225ca) and CClF₂CF₂CHClF (HCFC-225cb) using a Pd/C catalyst. Patent Document 4 listed below describes a process of coupling an alkane having 1 carbon atom with an alkane having 2 carbon atoms to produce an olefin having 3 carbon atoms such as HFC-1234yf. Patent Document 5 listed below describes a process of synthesizing an alkane having 3 carbon atoms by reacting an alkane having 1 carbon atom with an alkane having 2 carbon atoms, and dehyrohalogenating the synthesized alkane to produce a fluorinated olefin such as HFC-1234yf. Patent Document 6 listed below describes a process of passing CF₃CHFCHF₂ (HFC-236ea) and CF₃CHFCH₂F (HFC-245eb) simultaneously through a catalyst layer and subjecting them dehydrofluorination to directly produce a mixture of HFC-1225ye (1,1,1,2,3-pentafluoropropene) and HFC-1234yf. Patent Document 7 listed below describes a process of fluorinating CX₃CXYCH₃ with HF concurrently with a dehydrofluorination reaction to produce HFC-1234yf.
Patent Document 1: WO 2007/056194
Patent Document 2: U.S. Patent Application Publication No. 2007/0179324
Patent Document 3: WO 2007/086972
Patent Document 4: WO 2007/056127
Patent Document 5: WO 2007/056128
Patent Document 6: WO 2007/117391
Patent Document 7: WO 2007/056148
Patent Document 8: WO 93/25510

### Disclosure of Invention

However, these processes may often produce, in addition to HFC-1234yf as the aimed product and HFC-245eb as a precursor thereof, by-products which cannot be converted into HFC-1234yf. An object of the present invention is to provide a process capable of producing HFC-1234yf with a higher selectivity than that of the conventional methods.

The present inventors have studied about use of hexafluoropropene (HFP) as a raw material for production of HFC-1234yf, also intensively studied about appropriate procedure for carrying out hydrogenation, dehydrofluorination and distillation, and finally accomplished the present invention.

In a first aspect of the present invention, there is provided a process for producing 1,1,1,2-tetrafluoropropene (HFC-1234yf), which comprises the steps of:
a) hydrogenating a raw material of hexafluoropropene (HFP) together with 1,1,1,2,3-pentafluoropropene (HFC-1225ye) in the presence of a reducing catalyst to obtain a reaction mixture containing 1,1,1,2,3,3-hexafluoropropane (HFC-236ea) and 1,1,1,2,3-pentafluoropropane (HFC-245eb);
b) subjecting the reaction mixture obtained from the step a) to a dehydrofluorination reaction to obtain a reaction mixture containing 1,1,1,2,3-pentafluoropropene (HFC-1225ye) and 1,1,1,2-tetrafluoropropene (HFC-1234yf);
c) subjecting the reaction mixture obtained from the step b) to distillation to separate into a 1A fraction containing 1,1,1,2-tetrafluoropropene (HFC-1234yf) and a 2A fraction containing 1,1,1,2,3-pentafluoropropene (HFC-1225ye); and
d) using 1,1,1,2,3-pentafluoropropene (HFC-1225ye) contained in the 2A fraction for the step a),
whereby 1,1,1,2-tetrafluoropropene is obtained in the 1A fraction.

It is considered that, in the process according to the first aspect of the present invention, for producing HFC-1234yf from HFP, the following reactions successively proceed via HFC-1225ye.

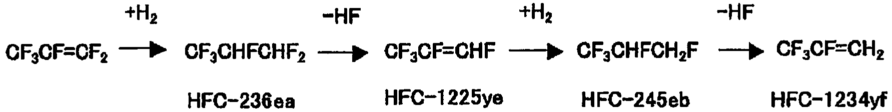

The present inventors have found that the hydrogenating reaction producing HFC-236ea from HFP and the hydrogenating reaction producing HFC-245eb from HFC-1225ye can proceed under the same conditions, and also the dehydrofluorination reaction producing HFC-1225ye from HFC-236ea and the dehydrofluorination reaction producing HFC-1234yf from HFC-245eb can proceed under the same conditions. Thus, the hydrogenations and the dehydrofluorinations are respectively carried out in the same times (in other words, by "folding" the above successive reactions), and then distillation is carried out so that HFC-1234yf is obtained. According to this process of the present invention, it becomes possible to produce HFC-1234yf with a higher selectivity.

In the process according to the above first aspect of the present invention,
the 2A fraction obtained from the step c) may contain, in addition to 1,1,1,2,3-pentafluoropropene (HFC-1225ye), at least one of 1,1,1,2,3-pentafluoropropane (HFC-245eb) and 1,1,1,2,3,3-hexafluoropropane (HFC-236ea); and
the step d) may comprise subjecting the 2A fraction to further distillation to separate into a 3A fraction containing at least one of 1,1,1,2,3-pentafluoropropane (HFC-245eb) and 1,1,1,2,3,3-hexafluoropropane (HFC-236ea) and a 4A fraction containing 1,1,1,2,3-pentafluoropropene (HFC-1225ye), subjecting the 3A fraction to the dehydrofluorination reaction of the step b) and subjecting the 4A fraction to the hydrogenating reaction of the step a).

Therefore, at least one of HFC-245eb and HFC-236ea as intermediates and HFC-1225ye can be used by returning to the each appropriate reaction stage.

However, the first aspect of the present invention is not limited thereto. For example, all of the 2A fraction as is may be subjected to the hydrogenating reaction of the step a).

### Brief Description of Drawings

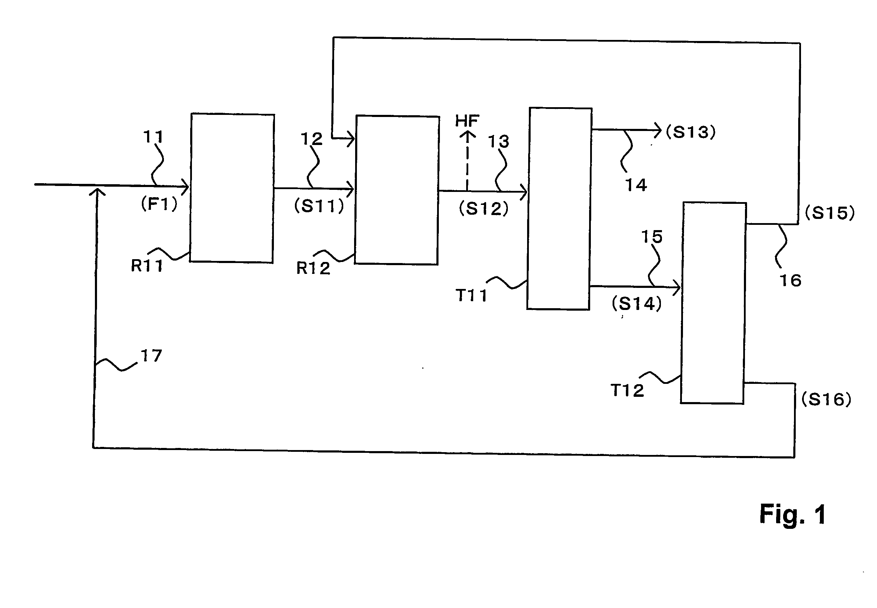

Fig. 1 shows a schematic diagram for explaining a process for producing 1,1,1,2-tetrafluoropropene (HFC-1234yf) in one embodiment of the present invention.

Following numerals denote the following elements: 11-17, ...line; R11, ...reactor (hydrogenating reaction); R12, ...reactor (dehydrofluorination reaction); T11, T12, ...distillation column; F1, ...feed (mixture); S11...first stream (reaction mixture); S12...second stream (reaction mixture); S13...third stream (1A fraction); S14...fourth stream (2A fraction); S15...fifth stream (3A fraction); 516...sixth stream (4A fraction);

### Best Mode for Carrying Out the Invention

An embodiments of the present invention will be described in detail below with reference to the drawing.

### (Embodiment 1)

The present embodiment relates to a manner in which following a hydrogenating reaction, dehydrofluorination is carried out, and then distillation is carried our ("folding" type).

Referring to Fig. 1, a raw material of hexafluoropropene (HFP) is fed from the outside to a line 11, and a sixth stream (S16) taken out of a distillation column T12 through a line 17 as mentioned below is recycled to the line 11. The sixth stream (S16) is a 4A fraction containing 1,1,1,2,3-pentafluoropropene (HFC-1225ye). Thus, the mixture of HFP and HFC-1225ye, as a feed (F1), is fed to a reactor R11 through the line 11.

The mixture of HFP and HFC-1225ye fed as the feed (F1) is subjected to hydrogenation in the presence of a reducing catalyst in the reactor R11 (in Fig. 1, supply of H₂ is omitted) to produce 1,1,1,2,3,3-hexafluoropropane (HFC-236ea) and 1,1,1,2,3-pentafluoropropane (HFC-245eb). In the reaction mixture, although the unreacted HFP and HFC-1225ye may slightly remain, it is preferred that there is substantially no material which would not be finally converted into HFC-1234yf.

As the reducing catalyst, for example, a general reducing catalyst can be used. A preferred reducing catalyst is a supported metal such as Pd, Pt, Rh, Ru or Re on activated carbon, a metal oxide such as alumina, or a metal fluoride. The ratio of metal supported is from 0.1 to 20% by weight, preferably from 0.1 to 10% by weight, and more preferably from 0.5 to 5% by weight.

Since ability of reduction varies depending on the kind of the catalyst and reduction may proceed excessively or may not proceed (a conversion ratio may not increase), the catalyst can be appropriately selected according to the purposes of the present embodiment.

The reaction pressure is not particularly limited and the reaction may proceed under reduced pressure, normal pressure or increased pressure, but the reaction under normal pressure or increased pressure is preferred. The pressure is preferably in a range from about 0 to 1.5 MPaG (gauge pressure), since such pressure has an advantage in that it does not require a comparatively large device for proceeding the reaction. However, it is also possible to apply a pressure out of the above range.

The reaction temperature is usually in a range from about 30 to 400°C, preferably from about 30 to 300°C, and more preferably from about 30 to 180°C. The amount of by-products can be suppressed to a comparatively small amount by the reaction temperature not being unnecessarily high. Since this reaction is an exothermic reaction, a temperature higher than the above range may occur locally (at a part of the catalyst).

The contact time is represented by W/F0 (g·Nml⁻¹·sec) (in this case, F0 (Nml·sec⁻¹) denotes the amount of the feed (F1) supplied to the reactor R11, and W (g) denotes the amount of the catalyst filling the reactor R11), it may be usually in a range from about 0.1 to 30, and is preferably in a range from about 0.5 to 15 (the symbol "N" in the unit means conversion into a normal condition of 0°C and 1 atm, the same shall apply hereinafter). The contact time exerts an influence on selectivity and conversion ratio, and therefore can be appropriately selected according to the purposes.

Hydrogen (H₂) used for hydrogenation is usually mixed with HFP as the raw material and HFC-1225ye in a ratio not smaller than a theoretically equivalent amount and supplied to the reactor R11. Therefore, the molar ratio in supply of H₂/(HFP and HFC-1225ye) is usually in a range from 1 to 6, and more preferably from 1 to 3.

Then, the reaction mixture obtained in the reactor R11 is taken out as a first stream (S11) through a line 12 and supplied to a reactor R12. And, HFC-236ea and HFC-245eb in the reaction mixture are subjected to dehydrofluorination to produce 1,1,1,2,3-pentafluoropropene (HFC-1225ye) and 1,1,1,2-tetrafluoropropene (HFC-1234yf). In a reaction mixture thus obtained, unreacted HFC-236ea, HFC-245eb and so on may remain.

For the dehydrofluorination reaction, a catalyst which can be used for a general dehydrohalogenation reaction can be used. The catalyst is specifically, for example, a metal oxyfluoride or a metal fluoride, and more specifically chromium oxyfluoride, aluminum oxyfluoride, niobium fluoride, magnesium fluoride, tantalum fluoride or antimony fluoride, but is not particularly limited thereto. For another example, an activated carbon based catalyst as disclosed in Patent Document 8 listed above may also be used.

The reaction pressure is not particularly limited and the reaction may proceed under reduced pressure, normal pressure or increased pressure, but the reaction under normal pressure is preferred. The reaction under normal pressure is advantageous in comparison to the reaction under increased pressure in view of equilibrium, and has an advantage in comparison to the reaction under reduced pressure in that it does not require a comparatively large device.

The reaction temperature, in a case under normal pressure, can be in a range from about 200 to 600°C, and preferably in a range from 250 to 450°C.

The contact time is represented by W'/F0' (g·Nml⁻¹ ·sec) (in this case, F0' (Nml-sec⁻¹) denotes the amount of the first stream (S11) and, if any, a below-mentioned fifth stream (S15) supplied to the reactor R12, and W' (g) denotes the amount of the catalyst filling the reactor R12) may be usually in a range from about 0.1 to 120, and is preferably in a range from about 0.5 to 60.

In the reactions of the present embodiment, the dehydrofluorination reaction is a rate-limiting step.

Then, the reaction mixture obtained in the reactor R12 after dehydrofluorination, is taken out as a second stream (S12) through a line 13 and supplied to a distillation column T11. The second stream (S12) contains HFC-1234yf (normal boiling point of about -29°C) and HFC-1225ye (normal boiling points of about -19.5°C (Z-isomer) and about -15.3°C (E-isomer)) and is separated by distillation into a 1A fraction containing HFC-1234yf (low-boiling component) and a 2A fraction containing HFC-1225ye (high-boiling component).

The 1A fraction is taken out of the distillation column T11 as a third stream (S13) through a line 14. The 1A fraction is preferably composed of HFC-1234yf substantially, and by the 1A fraction, HFC-1234yf can be obtained.

The 2A fraction is taken out of the distillation column T11 as a fourth stream (S14) through a line 15. The 2A fraction may generally contain, in addition to HFC-1225ye, HFC-245eb (normal boiling point of about 23°C) and/or HFC-236ea (normal boiling point of about 6°C).

The 2A fraction thus obtained after taking out of the distillation column T11 as the fourth stream (S14), is supplied to a further distillation column T12 and separated by distillation into a 3A fraction containing at least one of, generally both of, HFC-245eb and HFC-236ea (high-boiling components) and a 4A fraction containing HFC-1225ye (low-boiling component). The 3A fraction is taken out of the distillation column T12 through a line 16 as a fifth stream (S15), and may be returned to the reactor R12, where it is subjected to the dehydrofluorination reaction. The 4A fraction is taken out of the distillation column T12 through a line 17 as the sixth stream (S16), and supplied to the reactor R11 together with HFP as the feed (F1) as mentioned above, where it is subjected to the hydrogenating reaction.

As described above, according to the present embodiment, HFC-1234yf can be produced with a high selectivity by carrying out the successive reactions for obtaining HFC-1234yf from HFP through HFC-1225ye in the simplified process. The process of the present embodiment can be conducted continuously.

Although an amount of impurities produced in the present embodiment is quite small, low-boiling and high-boiling impurities may be removed from the stream(s) (e.g. the product stream and the recycling stream) by distillation, if appropriate. Hydrogen fluoride (HF) produced during the step may be separated and removed by washing with water and/or distillation, if appropriate. Although the unreacted hydrogen (H₂) may exist in the stream(s), it may be appropriately treated by, for example, separation and removal, or recycling to the reactor R11.

### [Example 1]

The process of the present invention was carried out in accordance with Embodiment 1 described in detail with reference to Fig. 1.

As a raw material, HFP was used in a gaseous state. This raw material and a sixth stream (S16) recycled from the distillation column T12 were combined and then fed, as a feed (F1), to the reactor R11, where the feed was subjected to the hydrogenation reaction in the presence of a reducing catalyst. As the reducing catalyst, an activated carbon-supported Pd catalyst (ratio of Pd supported: 3% by weight) was used. The reaction conditions were as follows: The reaction temperature was 200°C, and the reaction pressure was normal pressure. The volume ratio in supply of H₂/ (HFP and HFC-1225ye) was adjusted to 3. W / F0 = 8 (g·Nml⁻¹·sec), wherein F0 (Nml·sec⁻¹) denotes the amount of the feed (F1) supplied to the reactor R11, and W (g) denotes the amount of the catalyst filling the reactor R11.

The reaction mixture obtained in the reactor R11 was taken out as a first stream (S11) and supplied to the reactor R12, where it was subjected to the dehydrofluorination reaction. A fifth stream (S15) recycled from the distillation column T12 was also supplied to the reactor R12, and subjected to the dehydrofluorination reaction. As the catalyst, a chromium oxyfluoride catalyst (fluorine content: about 31.4% by weight) was used. The reaction conditions were as follows: The reaction temperature was 400°C, and the reaction pressure was normal pressure. W'/F0' = 20 (g·Nml⁻¹·sec), wherein F0' (Nml·sec⁻¹) denotes the amount of the first stream (S11) and the fifth stream (S15) supplied to the reactor R12, and W' (g) denotes the amount of the catalyst filling the reactor R12.

The reaction mixture obtained in the reactor R12 after the dehydrofluorination was taken out as a second stream (S12) and transferred to the distillation column T11, where it was separated into a third stream (S13) as a low-boiling part and a fourth stream (S14) as a high-boiling part. The resultant fourth stream (S14) was transferred to the distillation column T12, where it was separated into a fifth stream (S15) as a high-boiling part and a sixth stream (S16) as a low-boiling part. The resultant fifth stream (S15) and sixth stream (S16) were recycled entirely to the reactor R12 and the reactor R11 respectively, as described above.

Twelve hours after starting the operation, compositions of the feed (F1) and the first to sixth streams (S11 to S16) were analyzed by gas chromatography to determine the flow rate of each component excluding hydrogen (H₂) and hydrogen fluoride (HF). The results are shown in Table 1. In the table, the symbol "-" means no detection, and "trace" means existence only in a trace amount.

**Table 1**

| | Flow rate (kmol/hr) | | | | | | |
|---|---|---|---|---|---|---|---|
| | F1 | S11 | S12 | S13 | S14 | S15 | S16 |
| HFP | 0.51 | - | - | - | - | - | - |
| HFC-236ea | - | .51 | 0.08 | - | 0.08 | 0.08 | - |
| HFC-245eb | - | 0.49 | 0.05 | - | 0.05 | 0.05 | - |
| HFC-1234yf | - | - | 0.50 | 0.50 | - | - | - |
| HFC-1225ye | 0.49 | - | 0.49 | - | 0.49 | - | 0.49 |
| Impurities | trace | trace | 0.02 | 0.01 | 0.01 | 0.01 | trace |

It is confirmed from Table 1 that HFC-1234yf can be obtained as the third stream (S13), which corresponds to the 1A fraction, with a high yield.

### Industrial Applicability

1,1,1,2-tetrafluoropropene (HFC-1234yf) can be used as a refrigerant substance, and a process for producing HFC-1234yf using hexafluoropropene (HFP) as a raw material can be expected to be useful as a process for producing a substitute refrigerant.

## Claims

1. A process for producing 1,1,1,2-tetrafluoropropene, which comprises the steps of :
a) hydrogenating a raw material of hexafluoropropene together with 1,1,1,2,3-pentafluoropropene in the presence of a reducing catalyst to obtain a reaction mixture containing 1,1,1,2,3,3-hexafluoropropane and 1,1,1,2,3-pentafluoropropane ;
b) subjecting the reaction mixture obtained from the step a) to a dehydrofluorination reaction to obtain a reaction mixture containing 1,1,1,2,3-pentafluoropropene and 1,1,1,2-tetafluoropropene ;
c) subjecting the reaction mixture obtained from the step b) to distillation to separate into a 1A fraction containing 1,1,1,2-tetafluoropropene and 2A fraction containing 1,1,1,2,3-pentafluoropropene ; and
d) using 1,1,1,2,3-pentafluoropropene contained in the 2A fraction for the step a),
whereby 1,1,1,2-tetaftuoropropene is obtained in the 1A fraction.

2. The process according to claim 1, wherein the 2A fraction obtained from the step c) contains, in addition to 1,1,1,2,3-pentafluoropropene, at least one of 1,1,1,2,3-pentafluoropropane and 1,1,1,2,3,3-hexafluoropropane; and
the step d) comprises subjecting the 2A fraction to further distillation to separate into a 3A fraction containing at least one 1,1,1,2,3-pentafluoropropane and 1,1,1,2,3,3-hexafluoropropane and a 4A fraction containing 1,1,1,2,3-pentafluoropropane, subjecting the 3A fraction to the dehydrofluorination reaction of the step b), and subjecting the 4A fraction to the hydrogenating reaction of the step a).

3. Process according to claim 1 or 2 **characterised in that** the reducing catalyst is a supported Pd, Pt, Rh, Ru or Re on activated carbon, metal oxide or metal fluoride.

4. Process according to claim 3 **characterised in that** the ratio of metal supported is from 0.1 to 20% weight, preferably 0.1 to 10% weight and more preferably 0.5 to 5% weight.

5. Process according to any claim 1 to 4 **characterised in that** the reaction temperature of step (a) is from 30 to 400°C, preferably from 30 to 300°C and more preferably from 30 to 180°C.

6. Process according to any claim 1 to 5 **characterised in that** the catalyst in step (b) is a metal oxyfluoride, metal fluoride or activated carbon based.

7. Process according to any claim 1 to claim 6 **characterised in that** the reaction temperature of step (b) is from 200 to 600°C and preferably from 250 to 450°C.

## Patentansprüche

1. Verfahren zum Herstellen von 1,1,1,2-Tetrafluorpropen, umfassend die Schritte:
a) Hydrieren eines Ausgangsmaterials von Hexafluorpropen zusammen mit 1,1,1,2,3-Pentafluorpropen in Gegenwart eines reduzierenden Katalysators, um ein Reaktionsgemisch zu erhalten, das 1,1,1,2,3,3-Hexafluorpropan und 1,1,1,2,3-Pentafluorpropan enthält;
b) Unterwerfen des bei Schritt a) erhaltenen Reaktionsgemischs an eine Dehydrofluorierungsreaktion, um ein Reaktionsgemisch zu erhalten, das 1,1,1,2,3-Pentafluorpropen und 1,1,1,2-Tetrafluorpropen enthält;
c) Unterwerfen des bei Schritt b) erhaltenen Reaktionsgemischs einer Destillation, um es in eine Fraktion 1A, die 1,1,1,2-Tetrafluorpropen enthält, und eine Fraktion 2A, die 1,1,1,2,3-Pentafluorpropen enthält, zu trennen; und
d) Verwenden von 1,1,1,2,3-Pentafluorpropen, das in der Fraktion 2A enthalten ist, für den Schritt a),
wodurch 1,1,1,2-Tetrafluorpropen in der Fraktion 1A erhalten wird.

2. Verfahren gemäß Anspruch 1, wobei die bei Schritt c) erhaltene Fraktion 2A zusätzlich zu 1,1,1,2,3-Pentafluorpropen 1,1,1,2,3-Pentafluorpropan und/oder 1,1,1,2,3,3-Hexafluorpropan enthält; und
Schritt d) Unterwerfen der Fraktion 2A einer weiteren Destillation, um sie in eine Fraktion 3A, die 1,1,1,2,3-Pentafluorpropan und/oder 1,1,1,2,3,3-Hexafluorpropan enthält, und eine Fraktion 4A, die 1,1,1,2,3-Pentafluorpropan enthält, zu trennen, Unterwerfen der Fraktion 3A der Dehydrofluorierungsreaktion von Schritt b) und Unterwerfen der Fraktion 4A der Hydrierungsreaktion von Schritt a) umfasst.

3. Verfahren gemäß Anspruch 1 oder 2, **dadurch gekennzeichnet, dass** der reduzierende Katalysator ein getragenes Pd, Pt, Rh, Ru oder Re auf Aktivkohle, Metalloxid oder Metallfluorid ist.

4. Verfahren gemäß Anspruch 3, **dadurch gekennzeichnet, dass** der Anteil von getragenem Metall von 0,1 bis 20 Gew.-% beträgt, vorzugsweise 0,1 bis 10 Gew.-%, bevorzugter 0,5 bis 5 Gew.-%.

5. Verfahren gemäß einem der Ansprüche 1 bis 4, **dadurch gekennzeichnet, dass** die Reaktionstemperatur bei Schritt (a) 30 bis 400 °C beträgt, vorzugsweise 30 bis 300 °C, bevorzugter 30 bis 180 °C.

6. Verfahren gemäß einem der Ansprüche 1 bis 5, **dadurch gekennzeichnet, dass** der Katalysator bei Schritt (b) ein Metall-Oxyfluorid, Metallfluorid oder Aktivkohlebasierend ist.

7. Verfahren gemäß einem der Ansprüche 1 bis 6, **dadurch gekennzeichnet, dass** die Reaktionstemperatur bei Schritt (b) 200 bis 600 °C beträgt, vorzugsweise 250 bis 450 °C.

## Revendications

1. Procédé de production de 1,1,1,2-tétrafluoropropène, comprenant les étapes consistant à :
a) hydrogéner une matière première d'hexafluoropropène conjointement avec du 1,1,1,2,3-pentafluoropropène en présence d'un catalyseur réducteur, afin d'obtenir un mélange réactionnel contenant du 1,1,1,2,3,3-hexafluoropropane et du 1,1,1,2,3-pentafluoropropane ;
b) soumettre le mélange réactionnel obtenu à l'étape (a) à une réaction de déshydrofluoration afin d'obtenir un mélange réactionnel contenant du 1,1,1,2,3-pentafluoropropène et du 1,1,1,2-tétrafluoropropène ;
c) soumettre le mélange réactionnel obtenu à l'étape (b) à une distillation pour le séparer en une fraction 1A contenant du 1,1,1,2-tétrafluoropropène et une fraction 2A contenant du 1,1,1,2,3-pentafluoropropène ; et
d) utiliser le 1,1,1,2,3-pentafluoropropène contenu dans la fraction 2A pour l'étape (a)
ce par quoi du 1,1,1,2-tétrafluoropropène est obtenu dans la fraction 1A.

2. Procédé selon la revendication 1, dans lequel la fraction 2A obtenue dans l'étape (c) contient, outre le 1,1,1,2,3-pentafluoropropène, au moins un parmi le 1,1,1,2,3-pentafluoropropane et le 1,1,1,2,3,3-hexafluoropropane ; et
l'étape (d) comprend la soumission de la fraction 2A à une distillation supplémentaire afin de séparer en une fraction 3A contenant au moins un parmi le 1,1,1,2,3-pentafluoropropane et le 1,1,1,2,3,3-hexafluoropropane et une fraction 4A contenant du 1,1,1,2,3-pentafluoropropane, la soumission de la fraction 3A à la réaction de déshydrofluoration de l'étape (b), et la soumission de la fraction 4A à la réaction d'hydrogénation de l'étape (a).

3. Procédé selon la revendication 1 ou 2, **caractérisé en ce que** le catalyseur réducteur est Pd, Pt, Rh, Ru ou Re supporté sur charbon actif, un oxyde de métal ou un fluorure de métal.

4. Procédé selon la revendication 3, **caractérisé en ce que** le rapport de métal supporté va de 0,1 à 20% en poids, préférablement de 0,1 à 10% en poids et plus préférablement de 0,5 à 5% en poids.

5. Procédé selon l'une quelconque des revendications 1 à 4, **caractérisé en ce que** la température de la réaction de l'étape (a) va de 30 à 400°C, préférablement de 30 à 300°C, et plus préférablement de 30 à 180°C.

6. Procédé selon l'une quelconque des revendications 1 à 5, **caractérisé en ce que** le catalyseur dans l'étape (b) est un oxyfluorure de métal, un fluorure de métal ou basé sur charbon actif.

7. Procédé selon l'une quelconque des revendications 1 à 6, **caractérisé en ce que** la température de la réaction de l'étape (b) va de 200 à 600°C, et préférablement de 250 à 450°C.
